# EUROPEAN PATENT SPECIFICATION

(11) **EP 1 954 326 B1**
(45) Date of publication and mention of the grant of the patent: **25.04.2012**
(21) Application number: 06794765.5
(22) Date of filing: 16.10.2006
(51) Int. Cl.: A61L 9/03, A61L 9/02

(54) **AIR FRESHENER WITH ELECTRIC LIGHTING**
Luftbedufter mit Beleuchtung
Dispositif de rafraîchisseur d'air avec illumination

(30) Priority: 21.10.2005 GB 0521472
(43) Date of publication of application: 13.08.2008
(73) Proprietor: Reckitt Benckiser (UK) Limited, Slough, Berkshire SL1 3UH (GB)
(72) Inventor: GOREHAM, Philip, William, East Yorkshire HU17 7DW (GB); KENNEDY, David, Montvale, NJ 07654 (US); NEWTON, Paul, Hull HU8 7DS (GB)
(74) Representative: Carlin, Robert George
(86) International application number: PCT/GB2006/003819
(87) International publication number: WO 2007/045834

(56) References cited:
- WO-A-2005/074998
- WO-A2-2005/086245
- GB-A- 2 388 779
- JP-A- 2003 310 732
- US-B1- 6 779 905

## Description

The invention relates to a device for dispensing a fluid for fragrancing, deodorising or sanitising air. Particularly, the invention relates to an air freshening device incorporating a light.

Air freshener devices are commonplace, particularly in domestic environments where they are used to create a pleasant fragrance or mask a bad odour. The devices are generally of two types, a stand-alone type which can be placed away from a mains socket and which may be electrically operated by means of an electrical lead or batteries, or the type which plugs directly in to a mains socket, known as a plug-in device. Usually, either one of these said types operates in combination with a fragrance refill cartridge. The refill cartridge may be gel-based in which fragrance in a gel emanates on contact with air or, aerosol based, or alternatively, the refill cartridge may be wick-based, wherein a fragranced liquid is drawn up the wick and evaporates on exposure to the air.

WO2005074998 discloses a flameless candle that releases a volatile active, said candle comprising: at least one LED that emits a flickering light that emulates a flame of a candle; a container mount for receiving and securing a replaceable container containing a volatile active to be released into the ambient environment over time; and a support structure for supporting said at least one LED and said cartridge mount, wherein said support structure is configured to allow air flow across the replaceable container when the replaceable cartridge is mounted in said cartridge mount.

US6779905 relates to an apparatus and method for emitting light, sound and/or fragrance, which comprises a housing and means therein for connecting the apparatus to an electrical power source. A lighting system, sound system and fragrance system are mounted on or within the housing and are enabled simultaneously or individually by the electrical power source. The housing contains perforations, permeable membranes and/or translucent coverings on or in its walls to allow for the transmission of light, sound and fragrance when the apparatus is activated.

WO2005086245 discloses a conventional light bulb configured to mate with a light socket comprising a base also including a compartment for receiving and securing a replaceable scent cartridge.

There is a now a trend to make air freshener devices which are visually appealing to the consumer. However, such devices, although being eye-catching in shape, do not achieve the goal of creating an ambience in a room. Instead, the consumer often chooses to burn a traditional wax candle because the candlelight flicker produced is renowned for being mood enhancing.

Wax candles have a limited burning time. In particular, the initial visual effect of the burning candle is quickly diminished as the wax melts. In a relatively short period of time, the desired ambience in the room is lost and can only be remedied by the consumer lighting a fresh candle. The inherent disadvantage of burning candles is that they produce smoke which can tar the decoration of the room and they pose an obvious risk of causing a house fire. Due to the risk of fire, the consumer is advised not to leave burning candles unattended.

Wax candles can be fragranced but the consumer has limited control over the rate at which the fragrance emanates into the room. Further, the smoke produced from the candle can mask the fragrance.

It is an object of the present invention to provide a device for dispensing a fluid for fragrancing, deodorising or sanitising air which attempts to overcome the aforementioned problems.

According to a first aspect of the present invention there is provided a device for dispensing a fluid for fragrancing, deodorising or sanitising air, the device having a body comprising a shade, a chassis having a base, a cartridge housing mounted on the chassis and adapted for housing at least one cartridge containing a fluid to be dispensed, and a plurality of electrical light sources, characterised in that a heat source is located on the chassis adapted for accelerating the rate at which the fluid is released from the cartridge and the heat source is provided with a flange that extends outwardly from the heat source, and further that the plurality of electrical light sources are located on the base between the cartridge housing and a sidewall of the body and are located around a perimeter of the chassis.

Preferably, the sidewall of the body forms a part of the chassis. Alternatively, the sidewall of the body forms a part of the shade.

Preferably, the shade is curved such that light falling thereon from the or each electrical light source creates a flame-shaped patch of light on the shade.

Preferably, the shade is shaped to curve outwardly from the chassis and then inwardly towards a top portion of the cartridge housing. More preferably, the shade curves outwardly from a lower part of the chassis.

The arrangement provides a device which operates to dispense a fragrance, deodorising preparation or sanitising preparation into the air whilst also being capable of producing a lighting effect which is reminiscent of a candle flame flickering. In so doing, the device negates the need for burning a traditional wax candle. Advantageously, the device operates without the risks associated with burning candles and can therefore be left unattended in a room.

By locating the said light source on the base of the device, the candle flicker effect is visible from the base of the device.

The desired ambience in a room can be achieved easily and quickly by using the device. Furthermore, because the device can be kept running for a long period of time, the ambience is substantially sustainable, for example, throughout an evening. The desired flickering effect of the light on the body of the device, akin to a candle flame, is maintained until the consumer chooses to switch the light or device off.

Advantageously, the device simulates a wax candle and is capable of fragrancing a room. Unlike a fragranced wax candle which also produces smoke, the vapour from the device retains the fragrance throughout the emanation with no masking of fragrance occurring.

Preferably, the plurality of light sources are light emitting diodes having a light intensity of preferably about 500 to 10,000 milliCandelas (mcd), preferably about 3000 to 5000mcd, most preferably about 3900mcd. Such LEDs produce sufficient light to create the desired effect but at the same time require little electrical energy to work. Further, the LEDs do not heat up during use and are therefore a safe source of light to incorporate into a consumer product. Preferably the device incorporates a means for the consumer to adjust the intensity of the at least one light source. Further preferably, the device incorporates means whereby the brightness of the at least one light source is directly related to the intensity of fragrance dissemination.

The provided plurality of light sources produce a substantially uniform lighting effect around the device.

The LEDs mounted on the chassis produce a lighting effect which is effective around substantially 360° of the body of the device. In this manner, no matter which direction the consumer looks at the device, the same desired effect of the flickering candle flame is achieved.

Advantageously, the consumer does not need to place the device in a strategic position in the room. Instead, the choice of where to place the device is unrestricted, which is appealing to the consumer.

Most preferably, five light sources are provided and are preferably arranged in a symmetrical curved shape around the cartridge housing, preferably a circular arrangement. An especially preferred arrangement is horseshoe shape around the chamber.

This configuration has been found to be most effective for producing the desired result whilst keeping the number of LEDs to a minimum.

Preferably, each of the five light sources is a light emitting diode having a light intensity of preferably 500 to 10000mcd, preferably 3000-5000mcd, most preferably between 3800 to 4000mcd.

Preferably, a viewing angle of between approximately 5° and 180°, preferably 20° and 90°, most preferably substantially 50° is provided for each said light source.

The heat source produces heat which causes vaporisation of fluid in the device, for example a liquid fragrance, deodorising fluid, or sanitising fluid.

The light, in use, shines upwards towards the flange, to thereby project a shadow of the flange upwards towards a first end of the body.

The flange acts as a visor to minimise the shadowing effect of, for example, unsightly wiring inside the housing of the device. In particular, the flange acts to minimise the shadowing produced by light hitting a connector supplying power to the heat source. As a variation of this, the flange can be shaped so that themed shadows are produced from the light hitting the flange. The themed shadows may be projected on to the shade to be visible to the consumer. The shadows may be shaped like, for example, stars, or may include text to display a message.

Preferably, the cartridge housing has approximately the same dimensions to that of the cartridge. Preferably resilient means are located on the cartridge housing. Preferably, a plurality of cartridge housings is located on the chassis.

The cartridge housing is shaped to receive the cartridge in a snug releasable manner. Due to the shape matching, the cartridge can only be fitted into the cartridge housing in one orientation. Advantageously, the cartridge acts in a self guiding manner and therefore the onus of correctly fitting the cartridge is substantially removed from the consumer. The arrangement also helps to prevent damage to the wick of a device. This arrangement is thus suited to all ages and abilities of consumer.

Preferably, the cartridge housing has an upstanding collar which preferably extends upwards from the cartridge housing by a height of approximately 45mm and preferably, substantially vertically from said housing. The said collar preferably extends beyond the height of the heat source. Preferably, the collar is located between the chamber and the shade. Preferably, the maximum angle of the spread of the light generated from the front LED is such that the light from said LED substantially only casts a shadow on the collar which is not visible externally.

The collar prevents unsightly reflections, for example from wiring, appearing on the shade and marring the appearance of the device. Further, the collar controls the shadowing effect produced by the internal parts of the chassis. In particular, the collar acts as a reflector to reduce the shadows on the internal parts.

Preferably, the internal parts of the chassis and shade are smoothly contoured with no sharp corners or pointed edges. Further, snap fitting and screw points are smoothed. In this manner, the opportunity to create shadowing is minimised.

The use of a flange and smooth contours helps to control the shadowing effect produced by light reflecting off various parts of the internal surfaces of the shade and the chassis. By minimising the unwanted shadowing effect, the external device shows a clear image of the candle flame flicker effect.

Most preferably, parts of the body, preferably the cartridge housing, are manufactured from opaque material. The opaque material acts as a barrier to light shining from the light source and so further reduces the possibility of a shadowing effect.

Preferably, an image projecting portion is provided on the body which is preferably moveable with respect to the chassis or the shade. The image projecting portion is located on the body such that when light hits the image projecting portion, an image of the said portion is projected onto a surface of the body, preferably onto the shade. Provision of said portion is pleasing to the consumer. For example, if the device is being used in a restaurant, the name or the logo of the restaurant may be incorporated onto the image projecting portion. The name or logo will then be reflected onto the shade of the device. The reflection may be caused to rotate about the shade if the image projecting portion is rotatably mounted on the body. Preferably, a fragrance emanates from said device simultaneously of the candle flicker effect and the revolving image.

Preferably, the at least one electrical light source is operable to produce a visibly modulated light output in accordance with a selected mode of operation. The modulation of the light may be random, quasi-random, or pulsed in a sequenced fashion. Modulation means to vary the light output over a timescale so as to preferably change the colour of the LEDs over a period of time, preferably, over 20 seconds, more preferably over 15 seconds, most preferably over approximately 10 seconds. Preferably, speed control options are provided to set a cycle time between approximately 1 second and 60 seconds, preferably between 1.5 seconds and 50 seconds, most preferably between approximately 2.5 seconds and 40 seconds.

For example, when first switched on, the LEDs may glow yellow reminiscent of a cool flame. Over a period of time, for example, one hour, the LEDs may change colour to orange and then red. In this manner, the device would produce an effect akin to a burning flame, deepening in colour and intensity over time which would be considered to be more natural and mood enhancing than single colour LEDs which create the same lighting effect throughout. Preferably, each light emitting diode is of the same colour, preferably but not limited to yellow or orange. Alternatively, the colour may be selected from but not is necessarily limited to white, red, green or blue. Further preferably each LED is of a different colour to the neighbouring LED. Alternatively, each light emitting diode is an RGB type LED.

In most preferred arrangement, the cartridge is a wick based cartridge and the fluid is a fragrance which is vaporised by the heat source.

An embodiment of the invention will now be described, by way of example only, with reference to the accompanying drawings, wherein:
Figure 1 is a schematic perspective view of an air fragrancing device according to the invention;
Figure 2 shows a schematic sectional side view of an air fragrancing device incorporating a cartridge;
Figure 3 shows a schematic perspective view of a part of an air fragrancing device;
Figure 4 shows a schematic sectional view of an air fragrancing device;
Figure 5 shows a schematic plan view of a part of an air fragrancing device;
Figures 6a and 6b show schematic plan views of air fragrancing devices;
Figures 7a and 7b show further schematic plan views of air fragrancing devices;
Figures 8a and 8b show a schematic perspective view and a schematic sectional view respectively of an air fragrancing device;
Figures 9a and 9b show schematic sectional side views of air fragrancing devices; and
Figure 10 shows a schematic view from below an air fragrancing device.

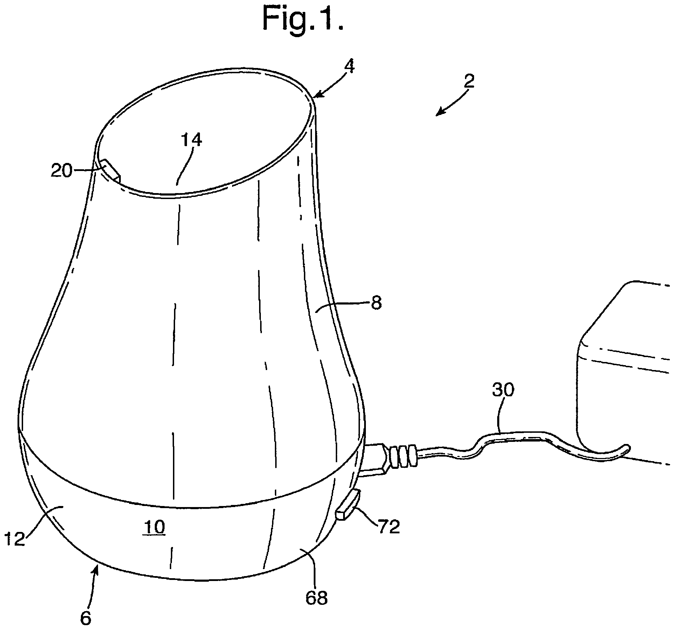

Figure 1 shows an air fragrancing device 2 according to the invention. The air fragrancing device 2 has generally a pear-shaped body having a chamfered first end 4 and a base 6. The air fragrancing device 2 comprises a shade 8 and a chassis 10. The shade 8 and the chassis 10 are held together in a push-fit manner but it will be appreciated that any fitting which allows the shade 8 to be removed from the chassis 10 would be equally suitable, for example, a screw thread type fitting.

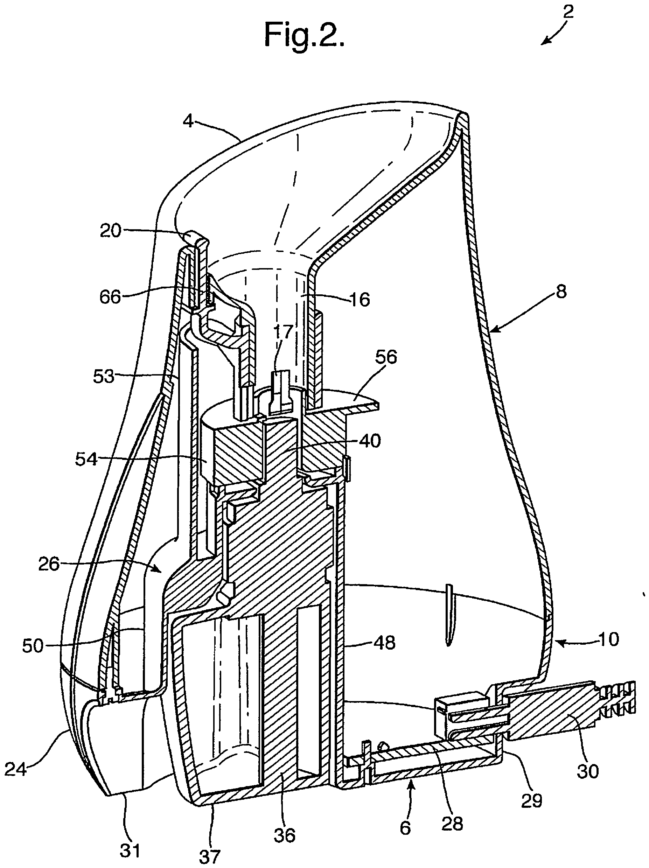

The shade 8 comprises the first end 4 and a second end 12. The first end 4 has a sloping surface in which is located an aperture 14 through which fluid exits the air fragrancing device 2. A channel 16 having a number of openings 17 therein extends downwardly from the aperture 14 towards the chassis 10 as shown in Figure 2. A switch 20 is located in a slot 22 in the first end 4. The slot 22 is of sufficient length to enable the switch 20 to move from at least a first position to at least a second position in order to effect the dispersion of the fluid. The switch 20 has a number of projections thereon which provide a gripping surface which a user of the air fragrancing device 2 can push against when moving the switch 20.

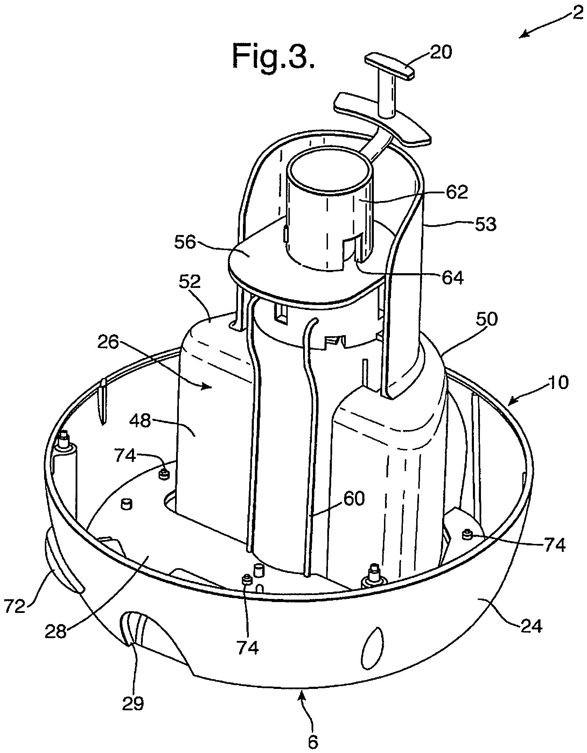

The chassis 10 is generally circular in plan view and comprises a skirt 24 and a chamber 26 as shown in Figures 2 and 3. A circuit board 28, as will be described in more detail below, is housed on the chassis 10. A cable opening 29 is located on the skirt 24 through which a cable 30 is fed into the device 2. The cable 30 connects to the circuit board 28 for supplying power to the air fragrancing device 2. The skirt 24 curves downwardly to join a support surface 31. The support surface 31 curves inwardly to form a part of the base 6.

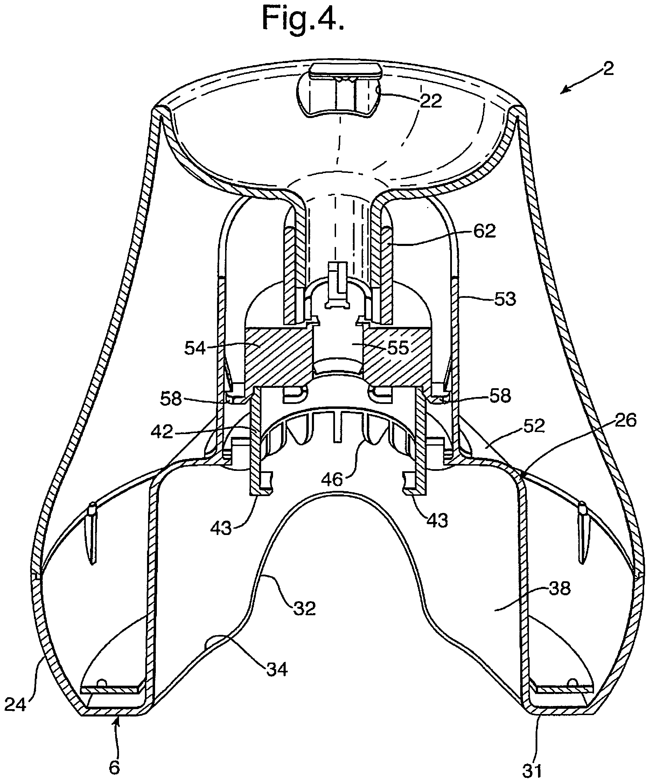

The skirt 24 has an arched vent 32 moulded therein as shown in Figure 4. The vent 32 extends underneath the chassis 10 to cooperate with an opening 34 in the support surface 31. During operation, the vent 32 enables a user to see a cartridge 36 in the device 2 and allows for easy insertion and removal of the cartridge 36.

A wall 38 extends upwardly from the opening 34 to form the chamber 26. The chamber 26 is of the same general shape to that of the cartridge 36 held therein. Due to this close similarity in shape the chamber 26 acts to guide the cartridge 36 into an operating position ready for use as shown in Figure 2.

The cartridge 36 is held in the chamber 26 such that a portion 37 of the cartridge 36 forms part of the base 6 of the device 2. The said portion 37 is coplanar with and adjacent to the support surface 31. As shown in figure 2, the portion 37 and the support surface 31 together form the base 6.

Resiliently deformable arms 42 are provided on an inner surface of the chamber 26. The arms 42 extend downwardly from the chamber 26. The arms 42 have hands 43 which project inwardly towards each other so that each hand 43 operates to lock the neck of the cartridge 36 in the operating position. A plurality of fins 46 are located at an upper end of the inner surface of the chamber 26 upon which shoulders of the cartridge 36 abut against when in the operating position. Seven fins 46 are shown but any number would be suitable. The fins 46 are arranged in a fan-like manner around the inner surface.

A typical cartridge 36 would be a wick-type liquid fragrance cartridge, commonly used as part of the AIRWICK (RTM) range. Although alternative cartridges include gel type cartridges, pressurised aerosol canisters, preferably incorporating a solenoid device in place of a heat source. The invention further includes passive air freshener devices, for example, those incorporating a fan and belt system.

As shown in Figure 2 and 3, the chamber 26 comprises a back portion 48 and a front portion 50 which are joined by a top portion 52. The chamber 26 is orientated so that the back portion 48 faces towards the cable 30. A collar 53 which is generally crescent-shaped in plan view extends around a part of the top portion 52, and upwardly towards the first end 4 of the air fragrancing device 2. The collar 53 extends from the top portion 52 to a point beyond the angle of spread of the light from, in particular the rear LED 80 as will be described in more detail below. The collar 53 prevents the light reflecting off the internal parts of the housing, for example, a heat source 54 or connector 60, from appearing on the first end 4.

The heat source 54 is located on the top portion 52 of the chamber 26. The heat source 54 is generally circular in cross section with a generally circular portal 55 extending through the length thereof. As shown in Figures 2 and 3, a flange 56 extends approximately 180° around an upper part of the heat source 54 and outwardly beyond the back portion 48. The preferred heat source 54 comprises two metal oxide resistors. The heat source operates at approximately 30 Ohms, 1.7 Watts, and 7.2 Volts. It will be appreciated by the skilled reader that any suitable heat source 54 could be incorporated into the air fragrancing device 2, for example, a wire-wound heater, a PTC, ceramic, a thick film or a thin film heater, or any other type of heater suitable for supplying heat to the wick 40.

The heat source 54 is held in position on the top portion 52 by lugs 58. The lugs 58 provide a snap fitting which allows for easy assembly of the heat source 54 on the chamber 26 during, for example, manufacture. The heat source 54 is connected to the circuit board 28 by a connector 60.

In an unclaimed arrangement not according to the invention, a chimney 62 is located on the heat source 54. The chimney 62 is generally circular in plan view and has an internal diameter of between 0.9 to 1.2cm. The length of the chimney 62 is approximately 2.5cm but may be up to 3cm in alternative embodiments. The chimney 62 has two diametrically opposing cut-outs 64 which are located at a lower end thereof. The cut-outs 64 are of similar dimension to the openings 17 in the channel 16. It is envisaged that any suitable number of cut-outs may be provided in the chimney 62. An arm 66 extends from the chimney 62 and through the slot 22 to cooperate with the switch 20. It will be appreciated by the reader that the switch 20 may either be a separate or integral part of the chimney 62. When the air fragrancing device 2 is assembled, the chimney 62 is held in position by the channel 16 which is of a slightly smaller diameter then said chimney. In this manner, the chimney 62 is movable relative to the channel 16 upon actuation of the switch 20.

Adjacent the cable opening 29 is an operating button 72 which is operatively linked to the circuit board 28. The operating button 72 is a push-button type, although a slidably movable operating button is equally suitable. A slidably movable operating button may be configured to move into a number of positions, which may be incremental.

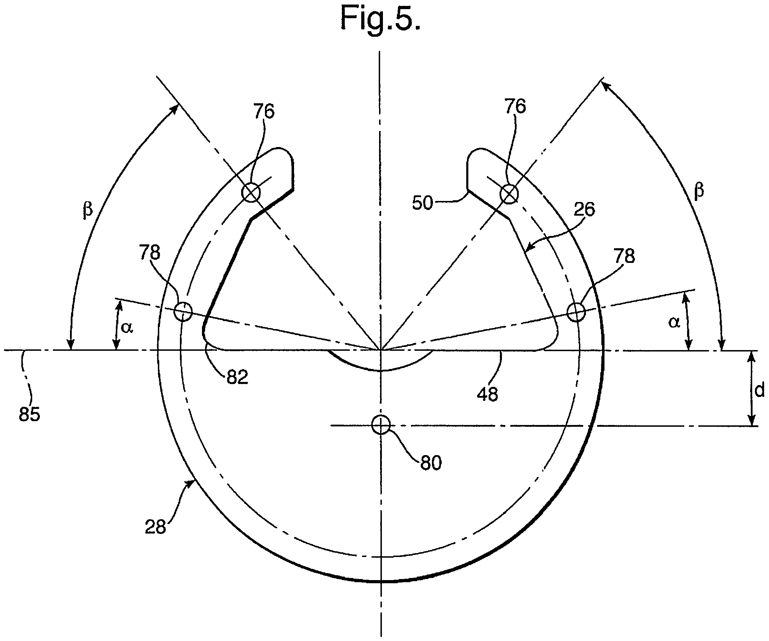

As shown in Figure 5, the circuit board 28 is generally horseshoe shaped in plan view, having a diameter of approximately 8cm. The circuit board 28 is connected to a power supply via the cable 30. The cable 30 connects to mains power through a transformer so as to supply low voltage electricity, typically 7.5V, to the air fragrancing device 2. In this manner, the user is not directly exposed to mains electricity.

A plurality of light sources shown as LEDs 74, are fixed on to the circuit board 28. Each LED 74 is fixed in a specific position. In the arrangement shown in Figure 5, five LEDs are provided and are configured in a symmetrical arrangement around the horseshoe shape of the circuit board 28 as follows: two front LEDs 76, two middle LEDs 78 and a single rear LED 80. The front LEDs 76 are positioned adjacent the front portion 50 of the chamber 26 at an angle β of approximately 49° from an axis 85. The middle LEDs 78 are positioned adjacent a corner 82 of the chamber 26, at an angle α which is approximately 11° from the axis 85. The rear LED 80 is positioned adjacent the back portion 48 of the chamber 26, and generally centrally thereof, at a distance d from the back portion 48. The distance d is approximately 0.14cm. In the arrangement shown, a 50° viewing angle is achieved for each LED 74. A 50° viewing angle means that the luminous intensity is half of the maximum at an angle 25° from the centreline of the LED 74. Although 50° is the preferred angle, the range of viewing angle includes 5 to 180°. The luminous intensity may be in the range 1000-5000mca but the preferred luminous intensity is 3900mca. The peak wavelength may be in a range of wavelength in the visible, UV or IR region but the preferred peak wavelength is 589nm. The spectrum radiation bandwidth may be between 5 to 50nm but in the embodiment shown, the preferred bandwidth is 30nm.

A filter element of substantially the same shape and size as the circuit board 28 is mounted on the chassis 10, above the LEDs and adjacent thereto. The filter element can rotate in the device 2. The filter element comprises a number of viewing windows, for example five windows. Each window is positioned to align with one LED 74 so that light passes through the or each window.

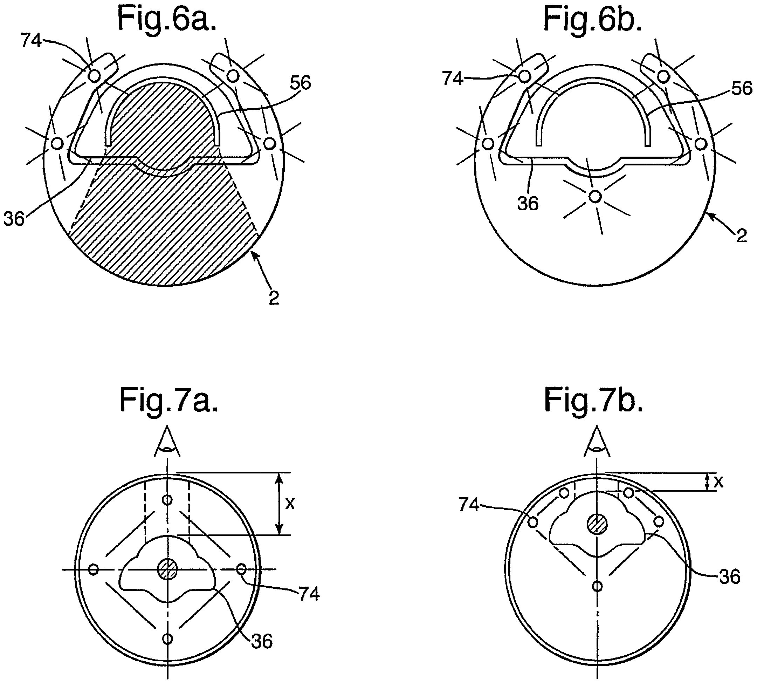

Figures 6a and 6b show alternative circuit boards having four or five LEDs respectively thereon. In the arrangement of Figure 6a, an unwanted shadow is created by the four LEDs as shown by hatched lines. In Figure 6b, five LEDs are located on a device. In this arrangement the shadowing in minimised.

Figures 7a and 7b again show arrangements for positioning either four or five LEDs respectively on the device. In Figure 7a, the position of the opening 34 in the base 6 of the device 2, together with the location and number of LEDs 74, reduces the visibility of the cartridge 36 when viewed through the vent 32. However, in the arrangement of Figure 7b, the visibility is substantially increased due to the location of the opening 34 and the number and positioning of each LED 74. The visibility distance X is reduced in Figure 7b which enables the consumer to easily see the cartridge 36.

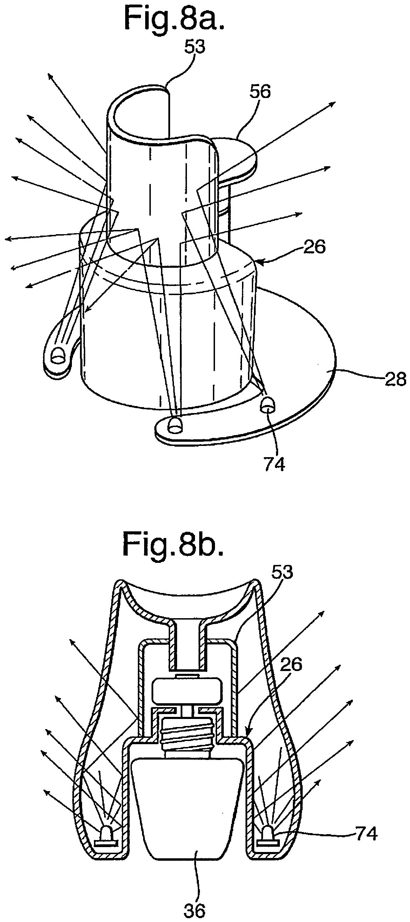

The positioning of the LEDs 74 with respect to the shade 8 and the chamber 26 provides an arrangement which produces a candle-like effect on the device 2. This effect is primarily due to the light from each LED 74 being reflected off the walls of the chamber 26 and the collar 53 and on to the inner surface of the shade 8 in a predictable and structured manner as shown in Figures 8a and 8b. The arrows in said figures schematically show the predicted direction the light travels in the device 2 and how the light is reflected off chosen surfaces.

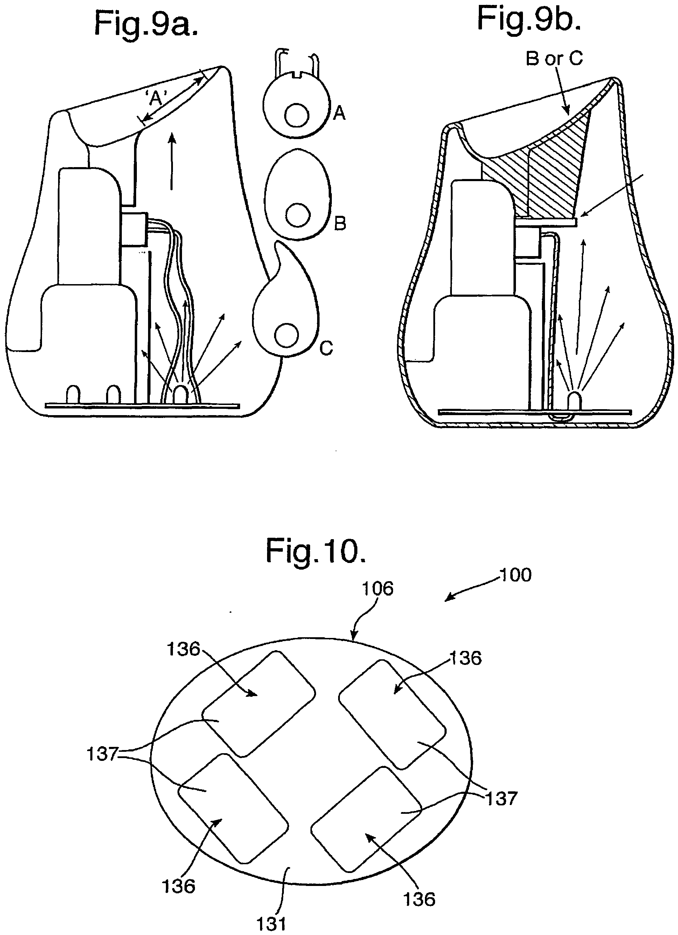

The arrangement provided by the invention overcomes problems of unwanted shadows being created from light reflecting randomly off component parts inside the device 2. An example of unwanted shadowing being created on the first end 4 of the device 2 is shown schematically in Figure 9a. In Figure 9a a shadow, shown as "A" in the figure, is created on the first end 4 due to light reflecting off the internal parts of the device 2, particularly the connector 60 and chamber 26. The effect "A" is undesirable to the consumer. Figure 9b shows the arrangement of the present invention whereby the flange 56 is provided on the heat source 54. The flange 56 extends passed the connector 60 and prevents light reflected off the connector 60 from being projected as a shadow on the first end 4. Instead, as shown by inserts marked B and C, desirable and visually appealing shadows are created on the first end 4. The shadows may be of any shape. For example, when used in a children's room, the shape may be a star shape and may be rotate about the aperture 14. This may be achieved by the flange 56 rotating in the device 2.

The LEDs 74 may be of the same colour or different colours. The colours are, for example, yellow, orange, white, red, blue or green. Alternatively at least one single RGB LED can be incorporated into the device 2. The light emitting from each LED 74 can be modulated. The alternative LEDs, together with modes of operation are detailed below.

There are a number of possible modes of operation of the LEDs 74. Each mode can be produced using RGB LEDs, or separate red, green and blue LEDs.

A first mode is known as the rainbow mode. The rainbow mode preferably cycles through all of the colours in a preferably continuous sequence over the course of approximately 10 seconds (s). Speed control options are provided on the device 2 to allow the consumer to set the cycle time, preferably to 2.5s, 5s, 10s, 20s or 40s or any time in between. In so doing, for example, a red LED 74 is slowly reduced in intensity while a blue LED 74 is slowly increased in intensity. The effect this produces is a gradual change through the colour spectrum from red, through purple to blue. The same effect is carried out with respect to a green LED 74 present in the device 2 so as to achieve a smooth cycle through the colour spectrum.

A second mode is the RGB beat mode which uses at least one RGB LED. The RGB beat mode cycles through the colours in preferably the same sequence as the rainbow mode, but the lights pulse off between each colour to give a beat effect. The "off cycle" is a gradual decrease then increase in light intensity over the course of approximately ls. The total colour cycle preferably takes 10s. Speed control options allow the consumer to set the cycle time a preferred time, for example, to 2.5s, 5s, 10s, 20s or 40s or any time in between. The interval of the "off cycle" is preferably varied proportionally with the cycle time.

Alternatively, the beat mode can be carried out on single LEDs 74. When using single LEDs, the consumer selects their chosen colour and the light pulses on/off in said colour. The total cycle time is preferably 6s. The "off cycle" is a gradual decrease then increase in light intensity over the course of preferably 1s. Speed control options allow the consumer to set the cycle time to a preferred time, for example, to 1.5s, 3s, 6s, 12s or 18s or any time in between. The interval of the "off cycle" is varied proportionally with the cycle time.

A further mode is the static mode. The static mode allows the consumer to select any of the possible colours to achieve a static light glow.

A further alternative mode is the pulse mode. The pulse mode is similar to the beat mode but the "off cycle" consists of preferably two off periods giving the effect of a heart beat. The total cycle time is preferably 8s. The light is fully on for 2s, a gradual loss in intensity over the course of 1s is then followed by a gradual increase over 1s. The light then gradually decreases in intensity over 1s and is held fully off for 1s before gradually increasing in intensity over 2s. Speed control options allow the consumer to set the preferred cycle time to 2s, 4s, 8s, 16s or 24s or any time in between. The interval of the off cycle is varied proportionally with the cycle time.

An RGB cycle mode gives the effect of colours moving around the device. In said mode, each RGB LED (or cluster of red, green and blue LEDs) is a different colour at any one time. For example, if three LEDs are used, one is red (LED1), one is green (LED2) one is blue (LED3) and at the start of the sequence. LED1 then slowly cycles from red to green, while LED2 cycles from green to blue and LED3 cycles from blue to red etc. The total cycle time is 10s. Speed control options allow the consumer to set the cycle time, for example, to 2.5s, 5s, 10s, 20s or 40s or any time in between.

A candle mode uses yellow LEDs or the yellow colour achieved by RGB LEDs. In said mode, one or more LEDs is held in a static yellow mode while one or more other LEDs is switched on/off in a yellow mode to produce a flicker effect. The on/off cycle is typically fast, with on and off being of preferably equal duration and preferably 5 cycles being achieved per second. The pulse speed can be varied by the consumer.

The mode of operation of the LEDs 74 can produce a flickering effect reminiscent of a candle flickering. Further, an effect may be produced over a programmed time period which may coincide with a change in the intensity of the fragrance. For example, a red candle may coincide with a strong intensity of fragrance and the fragrance decreases in intensity as the LED changes to an orange colour. The effect can be enhanced by the use of the filter element working in co-operation with the LEDs.

Figure 10 shows an alternative embodiment of the invention in which a plurality of cartridges 136 are housed in the chamber 126. Four cartridges 136 are shown but any suitable number may be incorporated into the device 100. A portion 137 of each cartridge 136 forms a section of the base 106 of the device 100, together with a support surface 131 of the device 100.

In a further embodiment, a circuit breaker is incorporated into the electrical circuitry. The circuit breaker can be operated to close the circuit when, for example, a fragrance cartridge is placed in the device 2 or the shade 8 is fitted to the chassis 10. In one embodiment, such circuit breaker may comprise a magnetic reed switch.

In a further embodiment, a recognition system is provided on the device 2. The recognition system comprises electronic identification means which can be located on the device 2, on each shade 8, and on a cartridge to be used in the device. The identification means detects a preferred match, for example, a particular shade with a particular fragrance cartridge. The operation of such a recognition system for example, incorporating barcodes, will be apparent to the skilled person.

In use, the consumer chooses a preferred shade 8 and fixes said shade 8 to the chassis 10. The consumer can make his choice using the recognition system. The cartridge 36 is then inserted into the opening 34 in the chamber 26 of the air fragrancing device 2. Advantageously, the vent 32 provides an easily accessible thumb hole for inserting and removing the cartridge 36. The contours of the chamber wall 38 act to guide the cartridge 36 towards an operative position in which the wick 40 passes through the chamber 26 and into the portal 55 of the heat source 54; the end of the wick 40 is co-linear with an end of the heat source 54. The shoulders of the cartridge 36 abut against the fins 46 of the chamber 26. The neck of the cartridge 36 is pushed against the arms 42 which resiliently deform to receive said cartridge 36. In this position, the air fragrancing device 2 is releasably held in the operative position.

In the operative position, the cartridge 36 sits in the device 2 so that the portion 37 of the cartridge 36 rests in the same plane as the support surface 31 of the chassis 10. Together, the portion 37 of the cartridge 36 and the support surface 31 form the base 6 of the device. Due to this configuration, a level base 6 of the device 2 is achieved. The user is able to place the device 2 down on a surface and the device remains in a steady configuration. In this position, a section of the cartridge 36 remains visible to the consumer due to the vent 32 in the skirt 24 of the chassis 10. This allows the consumer to monitor the level of liquid remaining in the cartridge 36, particularly when nearing the end of life of the cartridge 36.

In order to supply power to the air fragrancing device 2, the cable 30 is connected to a power supply. The cable 30 runs via a transformer which provides approximately 7.5V to said device 2. In so doing, the consumer is not directly exposed to the risk of viewing a mains electric shock. It will be appreciated that an alternative form of low voltage power supply may be incorporated, for example, low voltage battery power.

When connected, power is fed to the connector 60 to heat the heat source 54. Further, the LEDs 74 in the air fragrancing device 2 can be switched on by pushing the operating button 72 into an operative position. In an alternative arrangement, the operating button 72 can operate either or both the LEDs 74 or heat source 54.

When power is supplied to the heat source 54, heat radiates into the portal 55 to cause diffusion of the liquid on wick 40. When the switch 20 is in the 'open' position, the cut-outs 64 align with the openings 17 to allow a substantial flow of air across the end of the wick 40 which causes rapid vaporisation of the fragrance. Vapour travels up through the channel 16 and out of the aperture 14. If the user wishes to lessen the intensity of the fragrance the switch 20 is moved to a 'partially open' position. In this position, the chimney 62 rotates about the channel 16 so that the cut-outs 64 partially align with the openings 17 in the channel 16. Due to the gap provided between the openings 17 and cut-outs 64, a minimal but continual airflow is provided through the device 2 which helps to ensure that substantially no condensation forms on the internal surfaces of the device 2. The presence of condensation inside the device 2 is undesirable as liquid may run onto the electrical components, particularly the PCB, and damage the device 2. The air fragrancing device 2 is visually striking to a consumer while simultaneously operating as a fragrance dispenser. The light effect produced by light being directed towards particular surfaces on the chamber and shade, appears to the consumer as a candle flame, flickering in use and so there is no obvious requirement to burn wax candles which are potentially very dangerous. This effect is further enhanced by the consumer selecting a preferred mode of operation. Further, the light effect is not diminished by unsightly shadowing of internal parts of the device because of the arrangement of the collar 53 and flange 56 which co-operate to minimise such an effect. Also, the smoothed internal surfaces of the device 2 further ensure unwanted shadowing is minimised. Particularly, there are substantially no sharp corners on snap fits or screw points on the internal surface.

The ability to interchange shades 8 of the device 2 provides the consumer with a choice of external appearance of the device 2. Further, because a particular shade can be fitted with the identification means, the shade can be matched to a specific cartridge fragrance. The recognition means can be mechanical, for example, using co-operating mechanical parts on the shade and cartridge. The recognition system can also cause the operation of a particular mode of LEDs.

The vent 32 provides a viewing window for the consumer. The appeal of this feature is enhanced when the or each cartridge 36,136 is manufactured from transparent material and the fluid contained therein is coloured. When a plurality of cartridges is provided, each may contain a different coloured fluid. Also, due to the transparent nature of the or each cartridge, the consumer is given a visual indication of how full the cartridge is and when to replace it.

It is preferred that the or each cartridge is manufactured from glass. Glass is a robust material which can be blown thick enough to withstand impact. The manufacture of glass does not generally create sharp edges, which may sometimes result from injection moulding of plastics. Further, glass is compatible with the combination of fragrance and heat.

The configuration of a portion of the cartridge forming a part of the base of the device, together with the support surface of the chamber, requires less material to make the device. Therefore, the cost of producing the device is less than that compared to a device which incorporates a complete and integrally moulded base.

Provision of the component parts in the chassis 10 ensures that the operational parts are housed in one location on the device. This allows the shade to be manufactured more simply than if the shade carried component parts also. Further, because the shade has no component parts, the shade can be easily interchanged on the chassis by the consumer without the need for the consumer to connect any electrical components or other such parts which allow the device to operate. This arrangement provides the consumer with choice of device appearance but simultaneously limits the risk of the consumer damaging the device whilst fitting the chosen shade to the chassis.

The chamber may be manufactured from plastics material. Particularly suitable plastics materials are those which are opaque in nature, for example polypropylene. In so doing, the opaque areas aid in hiding internal component parts.

The invention is not limited to the use of a fragrance but is equally suited to use with liquids for pest control, pharmaceutical preparations which can be vaporised, for example, formulations to ease congestion. When used for pest control, the light source is preferably a UV light, particularly a UV germicidal light.

Although the device is shown connected to a mains power lead, it is envisaged that the device make comprise a docking station and rechargeable batteries. Such an arrangement allows the consumer to move the device around the home and outside, for example to the garden. When used in the garden, the device is preferably adapted for pest control.

## Claims

1. A device (2) for dispensing a fluid for fragrancing, deodorising or sanitising air, the device (2) having a body comprising a shade (8), a chassis (10) having a base (6), a cartridge housing (26) mounted on the chassis (10) and adapted for housing at least one cartridge containing a fluid to be dispensed, and a plurality of electrical light sources (74),
**characterised in that** a heat source (54) is located on the chassis (10) adapted for accelerating the rate at which the fluid is released from the cartridge and the heat source is provided with a flange (56) that extends outwardly from the heat source (54), and further that the plurality of electrical light sources (74) are located on the base (6) between the cartridge housing (26) and a sidewall of the body and are located around a perimeter of the chassis (10).

2. A device as claimed in any claim 1, wherein the shade (8) is curved such that light falling thereon from each electrical light source (74) creates a flame-shaped patch of light on the shade (8).

3. A device as claimed in any one of the preceding claims, wherein the shade (8) is shaped to curve outwardly from the chassis (10) and then inwardly towards a top portion of the cartridge housing (26).

4. A device as claimed in any one of the preceding claims, wherein a viewing angle of between 20° and 90° is provided by each light source (74).

5. A device as claimed in any one of the preceding claims, wherein a vent is provided on the chassis (10) and at least one of the electrical light sources (74) is located adjacent the vent.

6. A device as claimed in any one of the preceding claims, wherein the flange (56) extends substantially horizontally from the heat source (54).

7. A device as claimed in any one of the preceding claims, wherein the cartridge (36) is a wick-type cartridge and the fluid is a fragrance which is vaporised by the heat source (54) to produce a gas.

8. A device as claimed in any one of the preceding claims, wherein the cartridge housing (26) is manufactured from opaque material.

9. A device as claimed in any one of the preceding claims, wherein the electrical light sources (74) are operable to produce a visibly modulated light output in accordance with a selected mode of operation.

10. A device as claimed in any one of the preceding claims, wherein five light sources (74) are provided arranged in a symmetrical curved shape around the cartridge housing (26), preferably in a horseshoe-shape.

11. A device as claimed in claim 10, wherein the five light sources are LEDs having a light intensity of 500-10,000mcd.

## Patentansprüche

1. Vorrichtung (2) zum Abgeben eines Fluids, um Luft wohlriechend zu machen, von Gerüchen zu befreien oder hygienisch zu machen, wobei die Vorrichtung (2) einen Körper mit einer Abdeckung (8), einen Rahmen (10) mit einer Basis (6), ein Patronengehäuse (26), das an dem Rahmen (10) montiert ist und dazu ausgelegt ist, wenigstens eine Patrone aufzunehmen, die ein abzugebendes Fluid enthält, und mehrere elektrische Lichtquellen (74) umfasst,
**dadurch gekennzeichnet, dass** sich an dem Rahmen (10) eine Wärmequelle (54) befindet, die dazu ausgelegt ist, die Rate, mit der das Fluid von der Patrone freigegeben wird, zu erhöhen, und die Wärmequelle mit einem Flansch (56) versehen ist, der sich von der Wärmequelle (54) nach außen erstreckt, und dass sich ferner die mehreren elektrischen Lichtquellen (74) an der Basis (6) zwischen dem Patronengehäuse (26) und einer Seitenwand des Körpers befinden und längs eines Umfangs des Rahmens (10) angeordnet sind.

2. Vorrichtung nach Anspruch 1, wobei die Abdeckung (8) gekrümmt ist, derart, dass Licht, das von jeder elektrischen Lichtquelle (74) darauf fällt, auf der Abdeckung (8) einen flammenförmigen Lichtfleck erzeugt.

3. Vorrichtung nach einem der vorhergehenden Ansprüche, wobei die Abdeckung (8) so geformt ist, dass sie von dem Rahmen (10) nach außen gekrümmt ist und dann nach innen zu einem oberen Abschnitt des Patronengehäuses (26) gekrümmt ist.

4. Vorrichtung nach einem der vorhergehenden Ansprüche, wobei von jeder Lichtquelle (74) ein Betrachtungswinkel im Bereich von 20° bis 90° geschaffen wird.

5. Vorrichtung nach einem der vorhergehenden Ansprüche, wobei an dem Rahmen (10) ein Lüftungsloch vorgesehen ist und wenigstens eine der elektrischen Lichtquellen (74) sich in der Nähe des Lüftungslochs befindet.

6. Vorrichtung nach einem der vorhergehenden Ansprüche, wobei sich der Flansch (56) von der Wärmequelle (54) im Wesentlichen horizontal erstreckt.

7. Vorrichtung nach einem der vorhergehenden Ansprüche, wobei die Patrone (36) eine dochtartige Patrone ist und das Fluid ein Duftstoff ist, der durch die Wärmequelle (54) verdampft wird, um ein Gas zu erzeugen.

8. Vorrichtung nach einem der vorhergehenden Ansprüche, wobei das Patronengehäuse (26) aus einem lichtundurchlässigen Material hergestellt ist.

9. Vorrichtung nach einem der vorhergehenden Ansprüche, wobei die elektrischen Lichtquellen (74) betreibbar sind, um in Übereinstimmung mit einer gewählten Betriebsart eine Ausgabe von sichtbarem moduliertem Licht zu erzeugen.

10. Vorrichtung nach einem der vorhergehenden Ansprüche, wobei fünf Lichtquellen (74) vorgesehen sind, die in einer symmetrisch gekrümmten Form, vorzugsweise hufeisenförmig, um das Patronengehäuse (26) angeordnet sind.

11. Vorrichtung nach Anspruch 10, wobei die fünf Lichtquellen LEDs sind, die eine Lichtintensität von 500-10000 mcd haben.

## Revendications

1. Dispositif (2) servant à diffuser un fluide destiné à parfumer, désodoriser ou assainir l'air, le dispositif (2) comprenant un corps comportant un abat-jour (8), un châssis (10) comprenant une base (6), un logement (26) de cartouche monté sur le châssis (10) et conçu pour loger au moins une cartouche contenant un fluide destiné à être diffusé, et une pluralité de sources lumineuses électriques (74),
**caractérisé en ce qu'**une source (54) de chaleur est située sur le châssis (10) et conçue pour accélérer le débit auquel le fluide est libéré de la cartouche, et **en ce que** la source de chaleur est munie d'un flasque (56) qui s'étend en s'élargissant à partir de la source (54) de chaleur, et en outre **en ce que** la pluralité de sources lumineuses électriques (74) est située sur la base (6) entre le logement de cartouche (26) et une paroi latérale du corps, et est située autour d'un périmètre du châssis (10).

2. Dispositif selon la revendication 1, l'abat-jour (8) étant incurvé de telle sorte que la lumière projetée sur celui-ci en provenance de chaque source lumineuse électrique (74) crée une tache de lumière en forme de flamme sur l'abat-jour (8).

3. Dispositif selon l'une quelconque des revendications précédentes, l'abat-jour (8) présentant une forme telle qu'il s'incurve en s'élargissant à partir du châssis (10), puis en rétrécissant vers une partie supérieure du logement (26) de cartouche.

4. Dispositif selon l'une quelconque des revendications précédentes, un angle de vision compris entre 20° et 90° étant engendré par chaque source lumineuse (74).

5. Dispositif selon l'une quelconque des revendications précédentes, un évent étant pratiqué sur le châssis (10) et au moins une des sources lumineuses électriques (74) étant située au voisinage de l'évent.

6. Dispositif selon l'une quelconque des revendications précédentes, le flasque (56) s'étendant sensiblement horizontalement à partir de la source (54) de chaleur.

7. Dispositif selon l'une quelconque des revendications précédentes, la cartouche (36) étant une cartouche du type à mèche et le fluide étant un parfum qui est vaporisé par la source (54) de chaleur pour produire un gaz.

8. Dispositif selon l'une quelconque des revendications précédentes, le logement (26) de cartouche étant fabriqué à partir d'un matériau opaque.

9. Dispositif selon l'une quelconque des revendications précédentes, les sources lumineuses électriques (74) étant utilisables pour produire une sortie lumineuse visiblement modulée selon un mode de fonctionnement sélectionné.

10. Dispositif selon l'une quelconque des revendications précédentes, cinq sources lumineuses (74) étant installées, disposées suivant une forme incurvée symétrique autour du logement (26) de cartouche, de préférence suivant une forme de fer à cheval.

11. Dispositif selon la revendication 10, les cinq sources lumineuses étant des DEL présentant une intensité lumineuse de 500 à 10000 mcd.
